# EUROPÄISCHE PATENTSCHRIFT

(11) **EP 2 724 743 B1**
(45) Veröffentlichungstag und Bekanntmachung des Hinweises auf die Patenterteilung: **23.03.2016**
(21) Anmeldenummer: 13188544.4
(22) Anmeldetag: 14.10.2013
(51) Int. Cl.: A61M 16/08, A61M 16/20, A61M 16/06

(54) **Winkelstück für eine Beatmungsmaske**
Angled component for a breathing mask
Pièce d'angle pour un masque respiratoire

(30) Priorität: 25.10.2012 DE 102012020917
(43) Veröffentlichungstag der Anmeldung: 30.04.2014
(73) Patentinhaber: Drägerwerk AG & Co. KGaA, 23558 Lübeck (DE)
(72) Erfinder: Wallnewitz, Oliver, 23556 Lübeck (DE); Soltész, Krisztina, 23564 Lübeck (DE); Gawrysch, Alexandra, 27576 Bremerhaven (DE)

(56) Entgegenhaltungen:
- WO-A1-2005/051468
- WO-A2-2013/040198
- US-A- 5 647 355
- US-A1- 2010 154 799
- US-A1- 2012 055 471

## Beschreibung

Die vorliegende Erfindung betrifft ein Winkelstück gemäß dem Oberbegriff des Anspruches 1 und eine Beatmungssystem mit einem Winkelstück gemäß der vorliegenden Erfindung.

Für verschiedene medizinische Anwendungen ist eine künstliche oder unterstützende Beatmung von Patienten notwendig. Beatmungsgeräte dienen zur künstlichen Beatmung von Patienten. Bei einigen Beatmungsgeräten, die speziell im Bereich der Anästhesie eingesetzt werden, kann wenigstens teilweise das von dem Patienten ausgeatmete Exspirationsgas wieder als Inspirationsgas verwendet werden, d. h. es handelt sich um ein Rückatemsystem mit einem Atemluftkreissystem.

Die künstliche Beatmung von Patienten erfolgt dabei im klinischen Bereich oder auch bei einer Heimbeatmung. Hierzu ist das Atemgas durch wenigstens einen Beatmungsschlauch, insbesondere dem Inspirationsschlauch, dem Patienten zuzuführen. Dabei sind im Allgemeinen der Inspirations- und ein Exspirationsschlauch an einem Y-Stück angeschlossen und dieses ist wiederum mit einem Winkelstück verbunden. Durch das Winkelstück wird das Atemgas einer Beatmungsmaske zugeführt, welche auf den Gesichtsbereich des Patienten aufliegt. Das Winkelrohrstück begrenzt einen Atemgaskanal, durch welchen die Atemluft dem Patienten zugeführt und die ausgeatmete Luft abgeführt werden kann. An einem Schlauchanschluss des Winkelstückes kann ein Beatmungsschlauch oder das Y-Stück angeschlossen werden und an einem Maskenanschluss des Winkelstückes kann die Beatmungsmaske angeschossen werden. Das Winkelstück weist dabei ein Antierstickungsventil und Kohlendioxid-Ausatemöffnungen auf. Das Antierstickungsventil dient dazu, um ein Ersticken des Patienten, insbesondere bei einem Fehler oder einem Schaden an dem Beatmungsgerät zu verhindern. Durch das Antierstickungsventil kann somit der Patient direkt aus der Umgebung an dem Winkelstück Atemluft ansaugen. Mit den Kohlendioxid-Ausatemöffnungen soll verhindert werden, dass sich in Toträumen des Winkelstückes Kohlendioxid in größeren Konzentrationen ansammelt. Für unterschiedliche Anwendungen des Winkelstückes kann es dabei erforderlich sein, sowohl das Antierstickungsventil als auch die Kohlendioxid-Ausatemöffnungen geöffnet bzw. aktiviert zu haben oder nur das Antierstickungsventil geöffnet zu haben und die Kohlendioxid-Ausatemöffnungen geschlossen zu haben oder umgekehrt oder sowohl das Antierstickungsventil als auch die Kohlendioxid-Ausatemöffnungen geschlossen bzw. deaktiviert zu haben. Hierfür ist in nachteiliger Weise eine aufwendige Aktivierung und Deaktivierung mittels verschiedener Schaltorgane an dem Winkelstück erforderlich.

Aus der US 5 647 355 A ist ein Winkelstück mit einem Antierstickungsventil bekannt. Das Antierstickungsventil ermöglicht eine automatische Verbindung zu der Umgebungsluft bei einem Fehler und weist einen primären und sekundären Einlass sowie einen Auslass auf. Dieses Winkelstück aus US 5 647 355 A weist die Merkmale des Oberbegriffs von Patentanspruch 1 auf.

Die Aufgabe der vorliegenden Erfindung besteht deshalb darin, ein Winkelstück, für ein Maskensystem oder ein Beatmungssystem zur Verfügung zu stellen, welches bei einfacher Handhabung in verschiedenen Öffnungs- und Schließzuständen für das Antierstickungsventil und die Kohlendioxid-Ausatemöffnungen geschalten werden kann.

Diese Aufgabe wird gelöst mit einem Winkelstück, umfassend ein Winkelrohrstück, welches einen Atemgaskanal begrenzt, mit einem Schlauchanschluss mit einer Einlassöffnung zum Anschluss eines Beatmungsschlauches bzw. Y-Stückes und einem Maskenanschluss mit einer Auslassöffnung zum Anschluss der Beatmungsmaske, ein Antierstickungsventil, Kohlendioxid-Ausatemöffnungen, wobei das Winkelstück nur ein Schaltorgan zum Aktivieren und Deaktivieren des Antierstickungsventils und der Kohlendioxid-Ausatemöffnungen umfasst. Das Winkelstück weist nur ein Schaltorgan zum Aktivieren und Deaktivieren sowohl des Antierstickungsventils als auch der Kohlendioxid-Ausatemöffnungen auf. Dabei hat das Winkelstück ein erstes Betätigungselement an dem Antierstickungsventil zum Aktivieren und Deaktivieren des Antierstickungsventils durch Öffnen und Schließen einer Antierstickungsöffnung des Antierstickungsventils und ein zweites Betätigungselement an den Kohlendioxid-Ausatemöffnungen zum Aktivieren und Deaktivieren der Kohlendioxid-Ausatemöffnungen durch Öffnen und Schließen der Kohlendioxid-Ausatemöffnungen umfasst, und dass das erste und zweite Betätigungselement in mechanischer Wirkverbindung mit dem Schaltorgan stehen, so dass das erste und zweite Betätigungselement mittels des nur einen Schaltorganes jeweils zwischen einer Aktivierungsstellung und einer Deaktivierungsstellung bewegbar sind. Erfindungsgemäß ist vorgesehen, dass
das erste Betätigungselement als eine Klappe oder eine Membran ausgebildet ist,
an dem Schaltorgan ein Gabelteil befestigt ist, so dass das Gabelteil eine Bewegung des Schaltorganes mit ausführt und in der Aktivierungsstellung des ersten Betätigungselements kein Kontakt zwischen dem Gabelteil und der Klappe oder Membran besteht und in einer Deaktivierungsstellung des ersten Betätigungselements das Gabelteil in Kontakt mit der Klappe oder Membran steht und aufgrund des Kontaktes mit der Klappe oder Membran die Antierstickungsöffnung des Antierstickungsventils verschlossen ist,
das Schaltorgan als ein Ring ausgebildet ist und der Ring außenseitig an einem geraden Abschnitt des Winkelrohrstückes in Richtung einer Längsachse des geraden Abschnittes beweglich gelagert ist, der Ring als Drehring ausgebildet ist und um eine Drehachse verdrehbar ist, welche der Längsachse des geraden Abschnittes des Winkelrohrstückes entspricht.

Dadurch kann das Winkelstück in besonders einfacher Weise betätigt werden, da mit dem nur einen Schaltorgan sowohl das Antierstickungsventil als auch die Kohlendioxid-Ausatemöffnungen aktiviert und deaktiviert werden können, d. h. geöffnet und geschlossen werden können. Das Winkelstück ist damit in der Handhabung besonders einfach und komfortabel zum Öffnen und Schließen des Antierstickungsventiles und der Kohlendioxid-Ausatemöffnungen.

Das Schaltorgan ist ein Drehring. Ein Drehring an dem Winkelstück kann besonders einfach von einem Anwender des Winkelstückes betätigt werden.

Das erste Betätigungselement ist als eine Klappe oder eine Membran, insbesondere elastisch verformbare Membran, ausgebildet. Mittels einer Klappe oder Membran kann eine Antierstickungsöffnung an dem Winkelrohrstück besonders einfach geöffnet und geschlossen werden, so dass die Klappe oder die Membran als erstes Betätigungselement Bestandteil des Antierstickungsventiles ist.

An dem Schaltorgan, also dem Drehring, ist ein Gabelteil befestigt, so dass das Gabelteil ein Bewegung des Schaltorganes mit ausführt und in der Aktivierungsstellung des Gabelteils kein Kontakt zwischen dem Gabelteil und der Klappe oder Membran besteht und in der Deaktivierungsstellung des Gabelteils das Gabelteil in Kontakt mit der Klappe oder Membran steht und aufgrund des Kontaktes mit der Klappe oder Membran ist eine Antierstickungsöffnung des Antierstickungsventils verschlossen. Mittels des Gabelteils, welches fest mit dem Betätigungsorgan verbunden ist, kann die Klappe oder die Membran einfach zwischen der Aktivierungsstellung und der Deaktivierungsstellung bewegt werden.

Zweckmäßig ist das zweite Betätigungselement von dem Schaltorgan gebildet oder das zweite Betätigungselement ist von einer Klappe, einem Rollo oder einer Membran gebildet. Das zweite Betätigungselement kann von dem Schaltorgan selbst oder einem gesonderten Bauteil, z. B. einer Klappe, einem Rollo oder einer Membran, gebildet sein.

In einer weiteren Ausführungsform ist das Schaltorgan zwischen wenigstens drei verschiedenen Schaltstellungen beweglich gelagert.

Insbesondere sind in einer ersten Schaltstellung des Schaltorganes das Antierstickungsventil und die Kohlendioxid-Ausatemöffnungen aktiviert, in einer zweiten Schaltstellung des Schaltorganes ist das Antierstickungsventil aktiviert und die Kohlendioxid-Ausatemöffnungen sind deaktiviert und in einer dritten Schaltstellung des Schaltorganes ist das Antierstickungsventil deaktiviert und die Kohlendioxid-Ausatemöffnungen sind deaktiviert.

In einer weiteren Ausgestaltung sind in einer vierten Schaltstellung des Schaltorganes das Antierstickungsventil deaktiviert und die Kohlendioxid-Ausatemöffnungen aktiviert.

Der Drehring ist um eine Drehachse verdrehbar, welche der Längsachse des geraden Abschnittes des Winkelrohrstückes entspricht.
Erfindungsgemäßes Beatmungssystem, umfassend ein Beatmungsgerät, wenigstens einen Beatmungsschlauch, ein Winkelstück, eine Beatmungsmaske, wobei das Winkelstück als Winkelstück gemäß Patentanspruch 1 ausgebildet ist.

Zweckmäßig ist das Winkelrohrstück ein- oder mehrteilig.

Insbesondere wird nur eine Kohlendioxid-Ausatemöffnung, insbesondere eine große Kohlendioxid-Ausatemöffnung, als Kohlendioxid-Ausatemöffnungen betrachtet.

In einer weiteren Ausführungsform besteht das Winkelstück wenigstens teilweise, insbesondere vollständig, aus Kunststoff.

Es zeigt:
- Fig. 1: einen vereinfachte Darstellung eines Beatmungsgerätes,
- Fig. 2: einen Längsschnitt eines Winkelstückes bei einem aktivieren Antierstickungsventil und aktivierten Kohlendioxid-Ausatemöffnungen,
- Fig. 3: einen Längsschnitt eines Winkelstückes bei einem aktivieren Antierstickungsventil und deaktivierten Kohlendioxid-Ausatemöffnungen,
- Fig. 4: einen Längsschnitt eines Winkelstückes bei einem deaktivieren Antierstickungsventil und deaktivierten Kohlendioxid-Ausatemöffnungen,

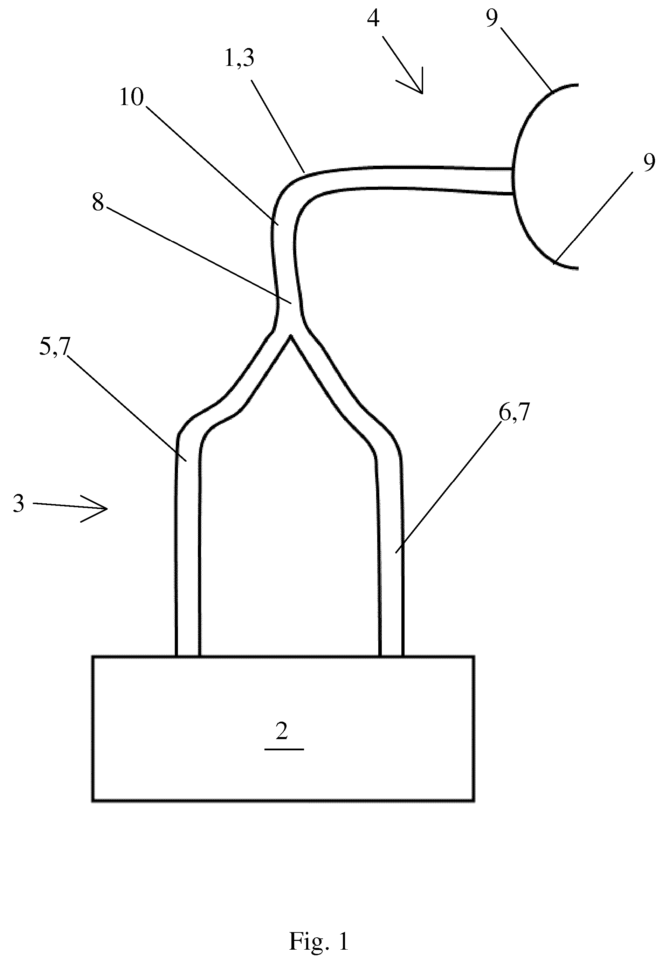

In Fig. 1 ist ein Beatmungsgerät 2 abgebildet.
Das dem zu beatmenden Patienten zugeführte Inspirationsgas wird dem Patienten durch einen Inspirationsschlauch 5 als Beatmungsschlauch 6 als Bestandteil eines Atemluftleitungssystems zugeführt. Nach dem Durchströmen des Inspirationsschlauches 5 und eines Y-Stückes 8 wird das Inspirationsgas durch ein Winkelstück 1 und einer Beatmungsmaske 9 dem Patienten zugeführt. Die von dem Patienten ausgeatmete Luft als Exspirationsgas wird durch einen Exspirationsschlauch 6 als Beatmungsschlauch 7 abgeleitet. Das Beatmungsgerät 2 bildet mit dem Beatmungsschläuchen 7, dem Y-Stück 8, dem Winkelstück 1 und der Beatmungsmaske 9 ein Beatmungssystem 3. Das Winkelstück 1 und die Beatmungsmaske 9 bilden ein Maskensystem 4.

Mit einem nicht dargestellten Stromanschlusskabel sowie einem Stromstecker kann das Beatmungsgerät 2 an das Stromnetz eines Krankenhauses angeschlossen werden. Außerdem weist das Beatmungsgerät 2 Gasversorgungsleitungen (nicht dargestellt) zum Anschluss an eine zentrale Sauer- und/oder Luft und/oder Lachgasversorgung des Krankenhauses auf.

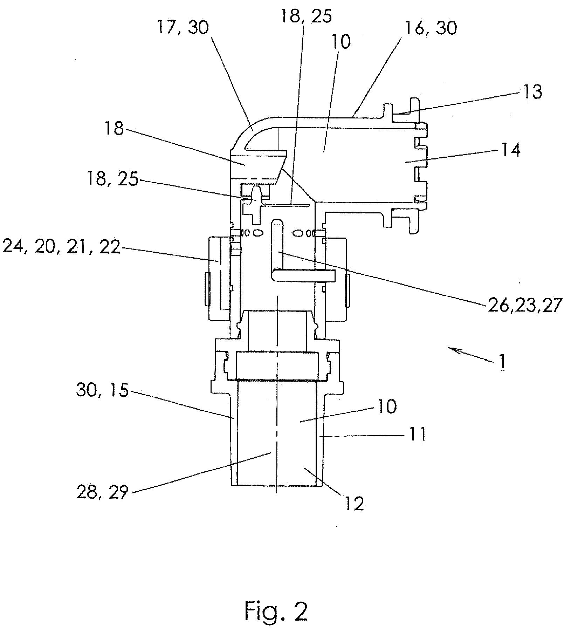

In den Fig. 2 bis 4 ist ein Längsschnitt des Winkelstückes 1 dargestellt. Das Winkelstück 1 weist ein mehrteiliges Winkelrohrstück 30 aus thermoplastischem Kunststoff auf, welches ein Atemgaskanal 10 zum Durchleiten von Exspirations- und Inspirationsgas bzw. Atemluft begrenzt. Das Winkelrohrstück 30 besteht aus einem ersten geraden Abschnitt 15 mit einer Einlassöffnung 12 zum Einleiten des Inspirationsgases und einem zweiten geraden Abschnitt 16 mit einer Auslassöffnung 14 zum Ausleiten des Inspirationsgases in die Beatmungsmaske 9. Die Ein- und Auslassöffnungen 12, 14 dienen ferner in umgekehrter Weise zum Durchleiten des Exspirationsgases. An dem ersten geraden Abschnitt 15 ist ein Schlauchanschluss 11 zum Anschließen bzw. Verbinden mit dem Y-Stück 8 bzw. einem Beatmungsschlauch 7 ausgebildet. In analoger Weise ist an dem zweiten geraden Abschnitt 16 ein Maskenanschluss 13 ausgebildet zum Anschließen bzw. Befestigen der Beatmungsmaske 9. Der erste und zweite gerade Abschnitt 15, 16 sind mit einem Bogen 17, welcher einer Krümmung vom im Wesentlichen 90° aufweist, miteinander verbunden. Der erste und zweite Abschnitt 15, 16 bzw. eine Längsachse 28 des ersten und zweiten geraden Abschnittes 15, 16, wobei nur die Längsachse 28 des ersten Abschnittes 15 dargestellt ist, stehen dadurch in einem im Wesentlichen rechten Winkel zueinander.

Das Winkelstück 1 ist ferner mit einem Antierstickungsventil 18 versehen. Das Antierstickungsventil 18 wird dabei von einer in den Fig. 2 bis 4 gestrichelt dargestellten Antierstickungsöffnung sowie einer Membran 25 gebildet. Mittels der Membran 25 kann die Antierstickungsöffnung des Antierstickungsventils 18 geöffnet und geschlossen werden. Bei einem geöffneten Antierstickungsventil 18 bzw. einer geöffneten Antierstickungsöffnung ist das Antierstickungsventil aktiviert und umgekehrt bei einem durch die Membran 25 geschlossenen Antierstickungsöffnung ist das Antierstickungsventil 18 deaktiviert. Das Antierstickungsventil 18 dient dazu, um bei einem Schaden oder einem Ausfall an dem Beatmungsgerät 2 weiterhin eine Beatmung des Patienten mit Luft aus der Umgebung zu ermöglichen. Mittels des Antierstickungsventils 18 kann unmittelbar durch das Winkelstück 1 Luft aus der Umgebung zur Beatmung des künstlich zu beatmenden Patienten zugeführt werden.

In Toträumen des Winkelstückes 1 kann sich Kohlendioxid ansammeln. Aus diesem Grund ist das Winkelstück 1 zusätzlich mit Kohlendioxid-Ausatemöffnungen 19 versehen. Durch die Kohlendioxid-Ausatemöffnungen 19 kann das Kohlendioxid in die Umgebung abgeleitet werden. Bei aktivierten Kohlendioxid-Ausatemöffnungen 19 sind diese geöffnet und bei deaktivierten Kohlendioxid-Ausatemöffnungen 19 sind diese geschlossen. Das Winkelstück 1 ist ferner nur mit einem Schaltorgan 20 sowohl zum Aktivieren und Deaktivieren des Antierstickungsventiles 18 als auch zum Aktivieren und Deaktivieren der Kohlendioxid-Ausatemöffnungen 19 versehen. Dabei ist das Schaltorgan 20 als ein Ring 21 ausgebildet, welcher auch einen Drehring 22 darstellt, da der Ring 21 nicht nur in Richtung eine Längsachse 28 des ersten geraden Abschnittes 15 beweglich mit einer Translationsbewegung gelagert ist, sondern auch um eine Drehachse 29 verschwenkbar bzw. rotationsbeweglich gelagert ist. Dabei entspricht die Drehachse 29 des Drehringes 22 der Längsachse 28 des ersten geraden Abschnittes 15 des Winkelrohrstückes 30. Der Drehring 22 ist dabei außenseitig an dem ersten geraden Abschnitt 15 des Winkelrohrstücks 30 ausgebildet.

Das Winkelstück 1 weist ferner ein erstes Betätigungselement 23 zum Aktivieren und Deaktivieren bzw. zum Öffnen und Schließen der Antierstickungsöffnung des Antierstickungsventils 18 auf. Das erste Betätigungselement 23 ist dabei von einer elastisch verformbaren Membran 25 gebildet. Das Winkelrohrstück 30 weist ferner eine Öffnung bzw. einen Schlitz auf, durch welchen ein Gabelteil 26 als Mechanismus 27 geführt ist und das Gabelteil 26 ist fest mit dem Drehring 22 verbunden.

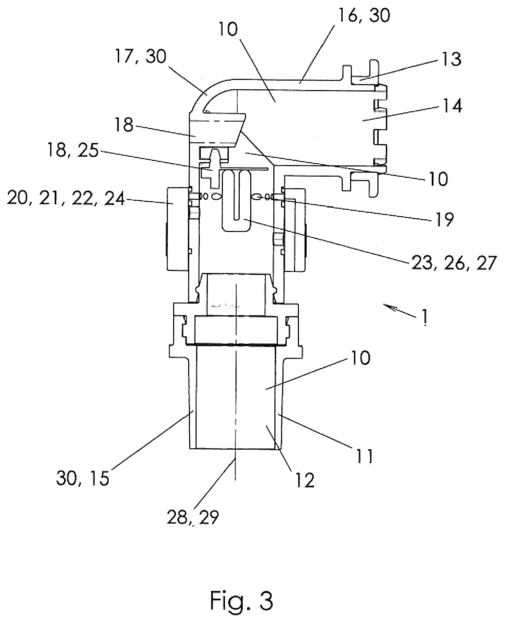

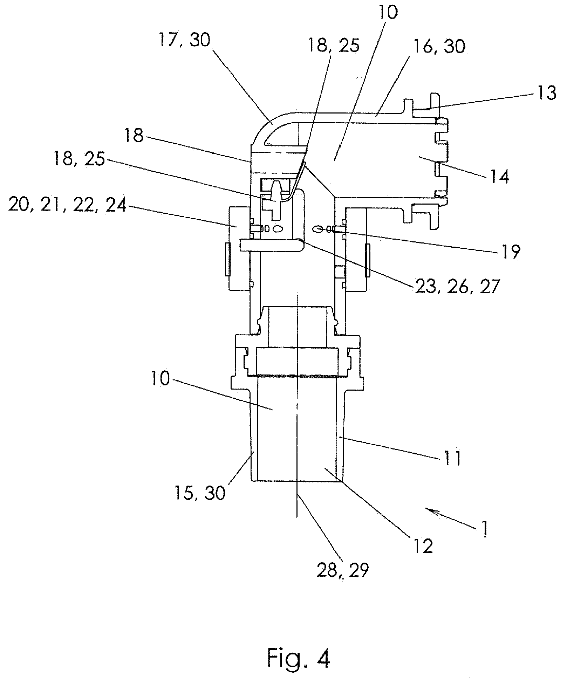

Der Drehring 22 als Schaltorgan 20 kann zwischen drei Schaltstellungen bewegt werden. In Fig. 2 ist eine erste Schaltstellung des Schaltorganes 20 dargestellt, bei welchem sowohl das Antierstickungsventil 18 als auch die Kohlendioxid-Ausatemöffnungen 19 aktiviert sind. In einer zweiten Schaltstellung des Schaltorganes 20, welche in Fig. 3 dargestellt ist, ist das Antierstickungsventil 18 aktiviert und die Kohlendioxid-Ausatemöffnungen 19 sind deaktiviert bzw. geschlossen. In einer dritten Schaltstellung des Schaltorganes 20 sind sowohl das Antierstickungsventil 18 als auch die Kohlendioxid-Ausatemöffnungen 19 geschlossen bzw. deaktiviert und diese Schaltstellung ist in Fig. 4 dargestellt. In der zweiten und dritten Schaltstellung des Drehringes 22 verschließt der Drehring 22 vollständig sämtliche Kohlendioxid-Ausatemöffnungen 19. Das Gabelteil 26 ist fest mit dem Drehring 22 verbunden und aufgrund der Translationsbewegung des Drehringes 22 in Richtung der Längsachse 28 steht das Gabelteil 26 in der zweiten Schaltstellung des Schaltorganges 20 in Kontakt mit der Membran 25. Das Schaltorgan 20, d. h. der Drehring 22, bildet auch ein zweites Betätigungselement 24 zum Öffnen und Schließen der Kohlendioxid-Ausatemöffnungen 19. In der in Fig. 3 und 4 dargestellten zweiten und dritten Schaltstellung des Schaltorganes 20 verschließt der Drehring 22 die umlaufend ausgebildeten Kohlendioxid-Ausatemöffnungen 19 vollständig.

Das Gabelteil 26 ist fest mit dem Schaltorgan 20 verbunden und bei einer Translationsbewegung des Schaltorganes 20 zwischen den drei Schaltstellungen führt auch das Gabelteil 26 eine Translationsbewegung in Richtung der Längsachse 28 des ersten Abschnittes 15 mit aus. In der ersten Schaltstellung des Schaltorganes 20 gemäß Fig. 2 besteht kein Kontakt zwischen dem Gabelteil 26 und der Membran 25. In der in Fig. 3 dargestellten zweiten Schaltstellung des Schaltorganes 20 besteht bereits ein Kontakt zwischen der Membran 25 und dem Gabelteil 26, jedoch ist die Membran 25 noch in einer Position, bei welcher die Antierstickungsöffnung nicht geschlossen ist. Bei einem weiteren Bewegen des Schaltorganes 20 von der zweiten in die dritte Schaltstellung bewegt sich auch das Gabelteil 26 gemäß der Darstellung in Fig. 3 und 4 nach oben, so dass dadurch die Membran 25 die Antierstickungsöffnung des Antierstickungsventils 18 verschließt.

Der Ring 21 als Drehring 22 ist ferner um die Drehachse 29 verschwenkbar. Mittels einer nicht gesondert dargestellten Kulissenführung und/oder dem Mechanismus 27, der nur teilweise als Gabelteil 26 dargestellt ist, kann das Schaltorgan 20 in den drei Schaltstellungen lösbar fixiert werden an dem übrigen Winkelstück 1 bzw. dem Winkelrohrstück 30. Die Herstellung dieser lösbaren Verbindung und das Lösen dieser lösbaren Verbindung erfolgt dabei jeweils zwischen den Schaltstellung durch ein Verschwenken des Drehringes 22 um die Drehachse 29.

Insgesamt betrachtet sind mit dem erfindungsgemäßen Winkelstück 1 wesentliche Vorteile verbunden. Das Winkelstück 1 ist mit nur einem Schaltorgan 20 als Drehring 22 versehen und mittels des nur einen Schaltorganes 20 können sowohl das Antierstickungsventil 18 als auch die Kohlendioxid-Ausatemöffnungen 19 aktiviert und deaktiviert werden. Dadurch ist das Winkelstück 1 in der Bedienung und Handhabung besonders einfach und praktisch. Es müssen somit nicht gesonderte Betätigungsorgane jeweils für das Antierstickungsventil 18 und die Kohlendioxid-Ausatemöffnungen 19 betätigt werden, sondern dies kann in einfacher und praktischer Weise mit dem nur einen Schaltorgan 20 ausgeführt werden.

### BEZUGSZEICHENLISTE

- 1: Winkelstück
- 2: Beatmungsgerät
- 3: Beatmungssystem
- 4: Maskensystem
- 5: Inspirationsschlauch
- 6: Exspirationsschlauch
- 7: Beatmungsschlauch
- 8: Y-Stück
- 9: Beatmungsmaske
- 10: Atemgaskanal
- 11: Schlauchanschluss
- 12: Einlassöffnung
- 13: Maskenanschluss
- 14: Auslassöffnung
- 15: Erster gerader Abschnitt mit Einlassöffnung
- 16: Zweiter gerader Abschnitt mit Auslassöffnung
- 17: Bogen
- 18: Antierstickungsventil
- 19: Kohlendioxid-Ausatemöffnungen
- 20: Schaltorgan
- 21: Ring
- 22: Drehring
- 23: Erstes Betätigungselement
- 24: Zweites Betätigungselement
- 25: Membran
- 26: Gabelteil
- 27: Mechanismus
- 28: Längsachse
- 29: Drehachse
- 30: Winkelrohrstück

## Patentansprüche

1. Winkelstück (1) für eine Beatmungsmaske (9) zur künstlichen Beatmung eines Patienten, umfassend
- ein Winkelrohrstück (30), welches einen Atemgaskanal (10) begrenzt, mit einem Schlauchanschluss (11) mit einer Einlassöffnung (12) zum Anschluss eines Beatmungsschlauches (7) bzw. Y-Stückes (8) und einem Maskenanschluss (13) mit einer Auslassöffnung (14) zum Anschluss der Beatmungsmaske (9),
- ein Antierstickungsventil (18),
- Kohlendioxid-Ausatemöffnungen (19),
wobei das Winkelstück (1) nur ein Schaltorgan (20) zum Aktivieren und Deaktivieren des Antierstickungsventils (18) und der Kohlendioxid-Ausatemöffnungen (19) umfasst, wobei das Winkelstück (1) ein erstes Betätigungselement (23) an dem Antierstickungsventil (18) zum Aktivieren und Deaktivieren des Antierstickungsventils (18) durch Öffnen und Schließen einer Antierstickungsöffnung des Antierstickungsventils (18) und ein zweites Betätigungselement (24) an den Kohlendioxid-Ausatemöffnungen (19) zum Aktivieren und Deaktivieren der Kohlendioxid-Ausatemöffnungen (19) durch Öffnen und Schließen der Kohlendioxid-Ausatemöffnungen umfasst, und dass das erste und zweite Betätigungselement (23, 24) in mechanischer Wirkverbindung mit dem Schaltorgan (20) stehen, so dass das erste und zweite Betätigungselement (23, 24) mittels des nur einen Schaltorganes (20) jeweils zwischen einer Aktivierungsstellung und einer Deaktivierungsstellung bewegbar sind,
**dadurch gekennzeichnet, dass**
das erste Betätigungselement (23) als eine Klappe oder eine Membran (25) ausgebildet ist,
an dem Schaltorgan (20) ein Gabelteil (26) befestigt ist, so dass das Gabelteil (26) eine Bewegung des Schaltorganes (20) mit ausführt und in der Aktivierungsstellung des ersten Betätigungselements kein Kontakt zwischen dem Gabelteil (26) und der Klappe oder Membran (25) besteht und in einer Deaktivierungsstellung des ersten Betätigungselements das Gabelteil (26) in Kontakt mit der Klappe oder Membran (25) steht und aufgrund des Kontaktes mit der Klappe oder Membran (25) die Antierstickungsöffnung des Antierstickungsventils (18) verschlossen ist,
das Schaltorgan (20) als ein Ring (21) ausgebildet ist und der Ring (21) außenseitig an einem geraden Abschnitt (15, 16) des Winkelrohrstückes (30) in Richtung einer Längsachse (28) des geraden Abschnittes (15, 16) beweglich gelagert ist, der Ring (21) als Drehring (22) ausgebildet ist und um eine Drehachse (29) verdrehbar ist, welche der Längsachse (28) des geraden Abschnittes (15, 16) des Winkelrohrstückes (3) entspricht.

2. Winkelstück nach Anspruch 1,
**dadurch gekennzeichnet, dass** das erste und/oder zweite Betätigungselement (23, 24) innenseitig an dem Winkelstück (1) angeordnet ist.

3. Winkelstück nach einem oder mehreren der Ansprüche 1 bis 2,
**dadurch gekennzeichnet, dass**
das zweite Betätigungselement (24) von dem Schaltorgan (20) gebildet ist oder das zweite Betätigungselement (24) von einer Klappe, einem Rollo oder einer Membran (25) gebildet ist.

4. Winkelstück nach einem oder mehreren der vorhergehenden Ansprüche,
**dadurch gekennzeichnet, dass**
das Schaltorgan (20) zwischen wenigstens drei verschiedenen Schaltstellungen beweglich gelagert ist.

5. Winkelstück nach Anspruch 4,
**dadurch gekennzeichnet, dass**
in einer ersten Schaltstellung des Schaltorganes (20) das Antierstickungsventil (18) und die Kohlendioxid-Ausatemöffnungen (19) aktiviert sind, in einer zweiten Schaltstellung des Schaltorganes (20) das Antierstickungsventil (18) aktiviert ist und die Kohlendioxid-Ausatemöffnungen (19) deaktiviert sind und in einer dritten Schaltstellung des Schaltorganes (20) das Antierstickungsventil (18) deaktiviert ist und die Kohlendioxid-Ausatemöffnungen (19) deaktiviert sind.

6. Winkelstück nach Anspruch 5,
**dadurch gekennzeichnet, dass**
in einer vierten Schaltstellung des Schaltorganes (20) das Antierstickungsventil (18) deaktiviert ist und die Kohlendioxid-Ausatemöffnungen (19) aktiviert sind.

7. Beatmungssystem (3), umfassend
- ein Beatmungsgerät (2),
- wenigstens einen Beatmungsschlauch (7),
- ein Winkelstück (1) und
- eine Beatmungsmaske (9),
**dadurch gekennzeichnet, dass**
das Winkelstück (1) gemäß einem oder mehreren der Ansprüche 1 bis 6 ausgebildet ist.

## Claims

1. An angled component (1) for a respiratory mask (9) for artificial respiration of a patient, comprising
- an angled tubular component (30), which defines a respiratory-gas channel (10), having a hose connection (11) with an inlet opening (12) for the connection of a respiratory hose (7) or Y-component (8) respectively, and having a mask connection (13) with an outlet opening (14) for the connection of the respiratory mask (9),
- an anti-suffocation valve (18),
- carbon-dioxide-expiration openings (19),
wherein the angled component (1) comprises only one switching part (20) to activate and deactivate the anti-suffocation valve (18) and the carbon-dioxide-expiration openings (19), wherein the angled component (1) comprises a first actuating element (23) at the anti-suffocation valve (18) to activate and deactivate the anti-suffocation valve (18) by opening and closing an anti-suffocation opening of the anti-suffocation valve (18) and a second actuating element (24) at the carbon-dioxide-expiration openings (19) to activate and deactivate the carbon-dioxide-expiration openings (19) by opening and closing the carbon-dioxide-expiration openings, and in that the first and second actuating element (23, 24) are operatively mechanically connected to the switching part (20) so that the first and second actuating element (23, 24) can be moved by means of the only one switching part (20) in each case between an activation position and a deactivation position,
**characterised in that**
the first actuating element (23) is formed as a flap or a membrane (25),
a fork portion (26) is secured to the switching part (20) so that the fork portion (26) also executes a movement of the switching part (20), and in the activation position of the first actuating element there is no contact between the fork portion (26) and the flap or membrane (25) and in a deactivation position of the first actuating element the fork portion (26) is in contact with the flap or membrane (25) and on account of the contact with the flap or membrane (25) the anti-suffocation opening of the anti-suffocation valve (18) is closed,
the switching part (20) is formed as a ring (21), and the ring (21) is movably mounted on the outside at a straight section (15, 16) of the angled tubular component (30) in the direction of a longitudinal axis (28) of the straight section (15, 16), the ring (21) is formed as a rotating ring (22) and can be turned about a rotational axis (29) which corresponds to the longitudinal axis (28) of the straight section (15, 16) of the angled tubular component (3).

2. An angled component according to claim 1,
**characterised in that**
the first and/or second actuating element (23, 24) are/is arranged on the inside at the angled component (1).

3. An angled component according to one or more of claims 1 to 2,
**characterised in that**
the second actuating element (24) is constituted by the switching part (20), or the second actuating element (24) is constituted by a flap, a shutter, or a membrane (25).

4. An angled component according to one or more of the preceding claims,
**characterised in that**
the switching part (20) is movably mounted between at least three different switching positions.

5. An angled component according to claim 4,
**characterised in that**
in a first switching position of the switching part (20) the anti-suffocation valve (18) and the carbon-dioxide-expiration openings (19) are activated, in a second switching position of the switching part (20) the anti-suffocation valve (18) is activated and the carbon-dioxide-expiration openings (19) are deactivated, and in a third switching position of the switching part (20) the anti-suffocation valve (18) is deactivated and the carbon-dioxide-expiration openings (19) are deactivated.

6. An angled component according to claim 5,
**characterised in that**
in a fourth switching position of the switching part (20) the anti-suffocation valve (18) is deactivated and the carbon-dioxide-expiration openings (19) are activated.

7. A respiratory system (3), comprising
- a respiratory device (2)
- at least one respiratory hose (7),
- an angled component (1), and
- a respiratory mask (9),
**characterised in that**
the angled component (1) is formed in accordance with one or more of claims 1 to 6.

## Revendications

1. Pièce coudée (1) pour un masque respiratoire (9) destiné à assurer la respiration artificielle d'un patient, comprenant
- une pièce tubulaire coudée (30), laquelle délimite un canal respiratoire (10), avec un raccord sur flexible (11) comprenant un orifice d'entrée (12), destiné à raccorder un flexible respiratoire (7), et avec une pièce en forme de Y (8) comprenant un raccord sur masque (13) avec un orifice de sortie (14), destiné à raccorder le masque respiratoire (9),
- une valve anti-étouffement (18),
- des orifices expiratoires (19) de dioxyde de carbone,
la pièce coudée (1) ne comprenant qu'un organe de commutation (20) destiné à activer et à désactiver la valve anti-étouffement (18) et les orifices expiratoires (19) de dioxyde de carbone, la pièce coudée (1) comprenant un premier élément de manoeuvre (23) sur la valve anti-étouffement (18), destiné à activer et à désactiver la valve anti-étouffement (18), par ouverture et fermeture d'un orifice anti-étouffement de la valve anti-étouffement (18) et un deuxième élément de manoeuvre (24) sur les orifices expiratoires (19) de dioxyde de carbone, destiné à activer et à désactiver les orifices expiratoires (19) de dioxyde de carbone, par ouverture et fermeture des orifices expiratoires de dioxyde de carbone, et le premier et le deuxième éléments de manoeuvre (23, 24) étant en liaison mécanique active avec l'organe de commutation (20), de sorte que le premier et le deuxième éléments de manoeuvre (23, 24) soient mobiles au moyen du seul organe de commutation (20), chacun entre une position d'activation et une position de désactivation,
**caractérisée en ce que**
le premier élément de manoeuvre (23) est conçu sous la forme d'un clapet ou d'une membrane (25),
sur l'organe de commutation (20) est fixée une pièce fourchue (26), de sorte que la pièce fourchue (26) accompagne un déplacement de l'organe de commutation (20) et dans la position d'activation du premier élément de manoeuvre, il n'existe aucun contact entre la pièce fourchue (26) et le clapet ou la membrane (25) et dans une position de désactivation du premier élément de manoeuvre, la pièce fourchue (26) soit en contact avec le clapet ou la membrane (25) et du fait du contact avec le clapet ou la membrane (25),
l'orifice anti-étouffement de la valve anti-étouffement (18) soit fermé,
l'organe de commutation (20) est conçu sous la forme d'une bague (21) et la bague (21) est logée sur une face extérieure d'une partie droite (15, 16) de la pièce tubulaire coudée (30), de manière mobile en direction d'un axe longitudinal (28) de la partie droite (15, 16), la bague (21) est conçue sous la forme d'une bague rotative (22) et peut tourner autour d'un axe de rotation (29), lequel correspond à l'axe longitudinal (28) de la partie droite (15, 16) de la pièce tubulaire coudée (3).

2. Pièce coudée selon la revendication 1, **caractérisée en ce que** le premier et/ou le deuxième élément de manoeuvre (23, 24) est placé sur la face intérieure de la pièce coudée (1).

3. Pièce coudée selon l'une quelconque ou plusieurs des revendications 1 et 2,
**caractérisée en ce que**
le deuxième élément de manoeuvre (24) est formé par l'organe de commutation (20) ou le. deuxième élément de manoeuvre (24) est formé par un clapet,
un store ou une membrane (25).

4. Pièce coudée selon l'une quelconque ou plusieurs des revendications précédentes,
**caractérisée en ce que**
l'organe de commutation (20) est logé en étant mobile entre au moins trois différentes positions de commutation.

5. Pièce coudée selon la revendication 4,
**caractérisée en ce que**
dans une première position de commutation de l'organe de commutation (20), la valve anti-étouffement (18) et les orifices expiratoires (19) de dioxyde de carbone sont activés, dans une deuxième position de commutation de l'organe de commutation (20), la valve anti-étouffement (18) est activée et les orifices expiratoires (19) de dioxyde de carbone sont désactivés et dans une troisième position de commutation de l'organe de commutation (20), la valve anti-étouffement (18) est désactivée et les orifices expiratoires (19) de dioxyde de carbone sont désactivés.

6. Pièce coudée selon la revendication 5,
**caractérisée en ce que**
dans une quatrième position de commutation de l'organe de commutation (20), la valve anti-étouffement (18) est désactivée et les orifices expiratoires (19) de dioxyde de carbone sont activés.

7. Système respiratoire (3) comprenant
- un appareil respiratoire (2),
- au moins un flexible respiratoire (7),
- une pièce coudée (1) et
- un masque respiratoire (9),
**caractérisé en ce que**
la pièce coudée (1) est conçue selon l'une quelconque ou plusieurs des revendications 1 à 6.
